(19) Europäisches Patentamt
European Patent Office
Office européen des brevets

(11) **EP 4 225 277 B1**

(12) **EUROPEAN PATENT SPECIFICATION**

(45) Date of publication and mention of the grant of the patent:
**13.05.2026 Bulletin 2026/20**

(21) Application number: **21805603.4**

(22) Date of filing: **07.10.2021**

(51) International Patent Classification (IPC):
***A61K 9/51*** (2006.01)

(52) Cooperative Patent Classification (CPC):
**A61K 9/5115**

(86) International application number:
**PCT/IB2021/059199**

(87) International publication number:
**WO 2022/074598 (14.04.2022 Gazette 2022/15)**

(54) **MESOPOROUS NANOPARTICLE SYSTEM TO INCREASE THE BIOFILM ERADICATION ACTIVITY BY MEANS OF THE CARRYING AND THE ADMINISTRATION OF AT LEAST ONE ACTIVE INGREDIENT AND RELATED PRODUCTION METHOD**

MESOPORÖSES NANOPARTIKELSYSTEM ZUR ERHÖHUNG DER BIOFILMAUSROTTUNGSAKTIVITÄT MITTELS TRÄGER UND VERABREICHUNG VON MINDESTENS EINEM WIRKSTOFF UND ZUGEHÖRIGES HERSTELLUNGSVERFAHREN

SYSTÈME DE NANOPARTICULES MÉSOPOREUSES POUR AUGMENTER L'ACTIVITÉ D'ÉRADICATION DE BIOFILM AU MOYEN DU TRANSPORT ET DE L'ADMINISTRATION D'AU MOINS UN PRINCIPE ACTIF ET PROCÉDÉ DE PRODUCTION ASSOCIÉ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **07.10.2020 IT 202000023665**

(43) Date of publication of application:
**16.08.2023 Bulletin 2023/33**

(73) Proprietor: **Brenta S.r.l.**
**36045 Lonigo (VI) (IT)**

(72) Inventors:
• **AMBROSI, Emmanuele Kizito**
**36075 Montecchio Maggiore (Vicenza) (IT)**
• **LEONETTI, Benedetta**
**36075 Montecchio Maggiore (Vicenza) (IT)**
• **CASTELLIN, Andrea**
**36075 Montecchio Maggiore (Vicenza) (IT)**
• **SPONCHIA, Gabriele**
**36075 Montecchio Maggiore (Vicenza) (IT)**

(74) Representative: **Feltrinelli, Secondo Andrea**
**APTA S.r.l.**
**Patent Department**
**Via Ca' di Cozzi, 41**
**37124 Verona (IT)**

(56) References cited:
**WO-A1-2016/204896     WO-A1-2017/041032**
**CN-A- 101 259 082**

• **SHAOHENG TANG ET AL: "Hollow Mesoporous Zirconia Nanocapsules for Drug Delivery", ADVANCED FUNCTIONAL MATERIALS, WILEY - V C H VERLAG GMBH & CO. KGAA, DE, vol. 20, no. 15, 9 August 2010 (2010-08-09), pages 2442 - 2447, XP001556978, ISSN: 1616-301X, DOI: 10.1002/ADFM.201000647**

**Description**

FIELD OF APPLICATION

The present invention is part of the technical sector of systems for the administration of substances inside the human body and has particularly as its object a system of nanoparticles for the administration and delivery of at least one active principle, generally known as DDS or drug delivery systems. Specifically, the invention relates to the use of a carrier which carries at least one active principle such as for example a substance having an antimicrobial and/or antibacterial and/or mucolytic nature for the eradication of biofilms, for example of bacterial origin.

TECHNICAL BACKGROUND

The methods for administering pharmaceutically and pharmacologically active ingredients show a wide variability in terms of technology and application, depending on some chemical and physical characteristics of the latter, such as solubility and permeability.

It is known that the most common methods of drug administration are by mouth or by injection. Lipophilic molecules represent a relatively difficult challenge in achieving systemic circulation. It is also known that these molecules belong, according to the Biopharmaceutical Classification System (BCS), to the second class (II), having low solubility and consequently low bioavailability (Advance Pharmaceutical Journal 2017; 2(6): 204-209).

In another context of the state of the art, it is known that microorganisms, primarily bacteria but also fungi, have a form of innate self-defense due to the formation of niches, called biofilms, which represent the most common condition of life, compared to the free or planktonic form (Pharmaceutics 2018, 10, 279).

The aforementioned biofilms are in fact consortia of microorganisms generally adhering to a biotic or abiotic surface, surrounded by a mucilaginous matrix that they self-produce. It is known that in this state, biofilms are more resistant to external attacks than cells in planktonic form.

Firstly, this is due to the presence of the matrix, an aqueous gel of exopolysaccharides, proteins and extracellular DNA that hinders the diffusion of molecules inside, secondly to the release of enzymes that degrade antibiotic molecules or to efflux pumps that extrude substances.

Furthermore, bacteria can live in a quiescent form such as persisters or small colony variants, with slowed metabolism.

From this it follows that conventional methods of treating biofilm-associated infections involve intensive administration of antibiotics, therefore high doses and for prolonged times. This generates numerous adverse effects, including emergence of antibiotic resistance and superbugs, adverse effects on the microbiome, or hypersensitivity.

These issues have led research to focus on alternative therapies to antibiotics, including bacteriophages, antimicrobial peptides, specific molecules that target structures within biofilms or that interfere with quorum sensing, i.e., the process that regulates communication between cells and which appears to be fundamental for the formation of the aforementioned structures such as biofilms.

In this context of innovation also nanotechnologies are inserted, structures of which at least one of the dimensions is of the order of nanometres and which, precisely because of the aforementioned dimensions, have peculiar characteristics different from the bulk counterpart of the same materials that constitute them.

Some nanoparticles (NPs) are studied precisely to have antibacterial characteristics, interacting with the bacterial membrane or wall, or generating reactive oxygen species or other nanostructures are able to deliver antibacterial drugs to the site of infection.

In this context, among the inorganic materials (Pharmaceutics 2018, 10, 279), mesoporous silica nanoparticles (MSN) emerge, known for their stability, high surface area and high pore volume, which give them high loading capacity of molecules of different types.

For this reason, the most recent studies have focused on the search for carriers that can overcome the aforementioned limit in the bioavailability of the so-called pharmaceutically active ingredients (API - Active Pharmaceutical Ingredients) (Nature Materials 2013 12: 991-1003; Expert Opinion on Drug Delivery 2016, 13(1): 93-108).

In this sense, the vehicle and drug delivery systems (DDS - Drug Delivery Systems) with a nanometric structure have aroused great interest as they provide large surface areas available for the adsorption of APIs.

Furthermore, they are versatile systems that allow multiple surface modifications to allow a more effective control of the release conditions for the molecules of interest.

Among the various solutions proposed, mesoporous inorganic materials have aroused particular interest, which have proved to be effective as API carriers.

Recent studies have focused in particular on the composition of mesoporous materials and on the main advantages deriving from mesoporous carriers based on the variation of structural parameters such as size, surface area, pore volume and pore distribution.

In particular, among these materials great attention has been paid to the use of mesoporous silica nanoparticles such as DDS, thanks to the ease of modifying the properties of these nanoparticles.

In fact, thanks to a surface area usually up to 1000 $m^2/g$ and a pore volume between 0.5-1.0 $cm^3/g$, these materials are able to accommodate molecules of different sizes, shapes and steric hindrance (Expert Opinion on Drug Delivery 2016, 13(1): 93-108; Pharmaceutics 2020, 12(5): 432; Molecules 2018, 23(1): 47).

Furthermore, the chemical-physical properties of these carriers, such as the surface charge or the presence of specific functional groups, can be suitably modified to specifically charge molecules with peculiar characteristics.

Last but not least, the possibility of functionalising both the internal and external surface of the nanoparticles with organic fractions, produces extremely versatile materials for further investigations, also in terms of the ability to interact with different biological effectors.

For example, surfaces can be rendered hydrophobic or hydrophilic, while the presence of organic residues could increase affinity for a specific molecular target or affect the bioavailability or biodistribution of nanoparticles.

Furthermore, different functionalizations could coexist on the same surface, expanding the field of possible applications.

In particular, it is known how titanium oxide or silicon dioxide nanoparticles could allow the prolonged and controlled release of drugs at the site of interest while preserving the stability of the charged molecules and improving their bioavailability for a prolonged time (Nanomed Nanotechnol 2018,3 (2):000136.; Acta Biomaterialia 2015 13: 354-363). Further advantages could be better solubility and permeability of biological membranes, better temporal stability and better therapeutic performance of drugs.

While mesoporous silica NPs such as DDS have been a highly studied research field due to their ease of modification of the properties of these nanoparticles (Pharmaceutics 2020, 12(5): 432), mesoporous zirconia ($ZrO_2$) nanoparticles (MZN) have been recently introduced and have been little studied (J. Mater. Chem.B, 2015, 3, 7300; WO2016120795; J. Drug Deliv. Sci. Technol, 2021, 61, 102189).

As is known, zirconium and its oxides are biocompatible and non-toxic materials and, for this reason, they are used in many applications, for example in orthopaedics and dentistry. Regarding zirconia nanoparticles, the toxicological profile is first strongly dependent on their size. For example, Yang et al., described intravenous administration of hollow $ZrO_2$ nanoparticles greater than 150 nm in diameter, which did not show toxicity up to 500 mg/kg in mice (International Journal of Nanomedicine 2019, 14: 5175-5186).

It follows that MZN particles could show important advantages for biomedical applications in vivo and for API loading processes.

From different references known to the literature, zirconium-based materials show a remarkable affinity for phosphate groups even in organophosphorus systems (Water, Air, & Soil Pollution 2012, 223: 4221-4231; Journal of the American Society for Mass Spectrometry 2008, 19(8): 1176-1186) and given the importance of the aforementioned functional groups in biological systems, it can be assumed that this characteristic of MZNs may play an important role in carrying molecules of interest.

WO2017/041032 discloses protocells including nanoparticles coated with a lipid layer; WO2016/204896 discloses drug-loaded mesoporous silica nanoparticles, wherein the nanoparticles are surface-functionalized with a plurality of aldehyde groups; CN101259082 discloses a root canal anti-inflammatory developer comprising titanium-stabilized mesoporous zirconia particles with an antibacterial anti-inflammatory drug attached thereto.

Therefore, we want to propose a new system for the delivery of substances, in particular pharmacologically active principles, which is proposed as a better alternative to the already known DDS and in particular to those using mesoporous silica nanoparticles, relying on a potential passive targeting system. intrinsic to the material, as well as stable and versatile.

OBJECTS OF THE INVENTION

The scope of the present invention is defined by the claims.

The purpose of the present invention is to provide a system for the administration and delivery of at least one active ingredient, in particular at least one drug of the type having antimicrobial and/or antibacterial and/or bacteriostatic and/or anti-inflammatory and/or mucolytic activity, also called DDS - Drug Delivery System, which is more efficient than traditional vehicle and administration methods and is proposed as an alternative to known systems, using vectors or carriers that allow to overcome the limits of the known ones. A particular object is to provide a system for the administration and the carrying of such active principles which have a better interaction with the bacterial or biofilm structures in order to be particularly effective against biofilm infections or contaminations.

A further purpose of the invention is to provide a system for conveying the aforementioned molecules, among which can be mentioned, in a non-exclusive way, antibacterial substances, antimicrobial substances, bacteriostatic substances, antibiotics, mucolytic substances, anti-inflammatory substances, etc.

Still another object is to provide a system for the vehicle and administration of drugs which can be obtained starting from carriers having greater availability and therefore greater cost-effectiveness.

These purposes are achieved by a vehicle or transport system for the administration of active substances, for example in particular drugs, which, according to claim 1, comprises a vector or carrier selected from the mesoporous nanoparticles having a surface provided with pores and one or more pharmacologically active ingredients (API) loaded and/or supported in the vector, in which said vector is constituted by mesoporous zirconia nanoparticles (MZN) and said at least one pharmacologically active ingredient wherein the API includes NAC.

A further object of the present invention is to provide a method for the production of mesoporous zirconia nanoparticles loaded with at least one API.

A still further object of the present invention consists in a system according to the present invention for use in the eradication of biofilm infections or contaminations (in which API is conveyed closed to and/or near and/or inside the biofilm itself).

BRIEF DESCRIPTION OF THE DRAWINGS

Other characteristics and advantages of the invention will be more evident from the description of an embodiment of a DDS system, illustrated by way of example in the accompanying drawings in which:

- figure 1 is a graph illustrating the log reduction and therefore the eradication of a *S. aureus* biofilm with respect to the concentration of NAC, in which the line with squares refers to free NAC while the line with circles refers to NAC loaded on MZN,
- figure 2 is a graph illustrating the logarithmic reduction and therefore the eradication of a *K. pneumoniae* biofilm with respect to the NTF concentration, in which the line with squares refers to free NTF while the line with circles refers to NTF loaded on MZN,
- figure 3 is a graph illustrating the logarithmic reduction and therefore the eradication of a *K. pneumoniae* biofilm with respect to the concentration of active ingredient (drug) or API, in which free NFT (NFT free), NTF loaded on MZN and the combination of NTF and NAC loaded on MZN is compared.

EMBODIMENTS OF THE INVENTION

In its most general form, a drug delivery system according to the invention, also called DDS - *Drug Delivery System,* comprises molecules having a vector or carrier function selected from mesoporous nanoparticles, in particular mesoporous zirconia nanoparticles MZN and one or more pharmacologically active ingredients, or API - *Active Pharmaceutical Ingredient,* loaded and/or supported in the nanoparticles of MZN.

The invention comprises a system of mesoporous zirconia nanoparticles MZN loaded with at least one API effective against biofilm infections or contaminations, wherein said API includes N-acetyl-L-cysteine (NAC) and which can - as better explained below - for example act as a so-called "Trojan horse" towards the biofilm itself.

The mesoporous zirconia nanoparticles comprise an internal structure and an external surface and the pores of such mesoporous nanoparticles are present in this internal structure and/or in this external surface.

Mesoporous zirconia nanoparticles (MZNs) loaded with an API, or for example an antimicrobial substance, can permeate the barrier constituted by the biofilm, in particular for example by the exopolymeric substances (EPS) that constitute it, and thus release the API or the antimicrobial substance close to and/or near the bacteria or microorganisms that populate the biofilm. This could depend, in at least one version, on the fact that MZNs can interact directly with the bacterial cell wall thanks to the high affinity of zirconia with phosphate groups.

In one version of the invention, the API can be loaded and/or supported inside the pores, in another version, the at least one API can be adsorbed on the outer surface of the nanoparticles, in a still further version, both possibilities can be present.

In this way, it is possible to overcome at least one of the reasons why biofilms show resistance to antibiotic therapies: as mentioned, in fact, the barrier constituted by the biofilm, and/or in particular by its exopolymeric substances, creates an obstacle to antimicrobial agents, which are delayed by the presence of this structure or neutralized/inactivated by it.

Therefore, the fact that MZNs can cross the barrier would allow to overcome the resistance mechanisms put in place by bacteria and by the biofilm it produces. Furthermore, this facilitates the penetration of the API, both by exploiting the water flow channels and by adhering to the biofilm barrier, as well as by hiding the API or antibiotic substance from enzymes produced by the biofilm itself, efflux pumps and other mechanisms put in place for the inactivation of these substances.

The first experimental tests demonstrated the particular effectiveness of using MZN as a loading system for the indicated APIs: in the experimental conditions used, the APIs proved to be stable and the MZNs inert in terms of reactivity towards the molecules examined.

Therefore, in at least one version of the invention, the dose-dependent adverse effects of at least one API are reduced when such agent is loaded and/or supported in the MZNs.

The release process was complete within 24 hours for the loaded and/or supported APIs, making the molecules available to explicit their mechanism of action, particularly improving the eradication action of biofilms.

Furthermore, mesoporous zirconia nanoparticles were found to be inert towards bacteria if not loaded with the API both by antibacterial tests on planktonic strains and by antibiofilm tests on sessile counterparts.

The eradication of preformed biofilms shows an increase in the antibiofilm activity of the API if the latter is loaded and/or adsorbed in the mesoporous nanoparticle and/or supported by the vector. In this state, the API is in fact protected and is released close to and/or near the microbial cells, thus performing improved performances. As a result, the amount of API, required to eradicate biofilm infection or contamination is reduced compared to other methods.

Specifically, at least one of the APIs is including N-acetylcysteine or NAC.

It is in fact known that NAC, or N-acetylcysteine, has antioxidant and mucolytic properties and is available on the market in various formulations (PCT/EP2017/082512; WO2018/154091 A1); vancomycin, VCN, is a glycopeptide antibiotic, known to be effective against various pathogenic strains including staphylococci (Scientific Reports 2020 10: 960); Nitrofurantoin (NTF) is a nitrofuran class antibacterial known for therapies for urinary tract infections (Clinical Microbiology and Infection 2017, 23(6): 355-362).

These APIs may be used either individually or in combination with each other or in combination with one or more APIs chosen from those not listed above.

In particular, the combinations can provide for the loading and/or support of more APIs in the same MZN or the combination and/or mixing of MZNs loaded with and/or supporting a first API with MZN loaded with and/or supporting a second API (where the first API is different from the second API), and so on.

The carrier made up of mesoporous zirconia nanoparticles, in fact, is so versatile that more APIs with different properties can be combined and mixed together to improve their overall activity and/or, possibly, to obtain the same activity using a smaller amount of APIs.

However, in the choice of several APIs it is necessary to exclude, or at least carefully evaluate, those combinations that can lead to an antagonism of the active ingredients loaded and/or supported in the same mesoporous zirconia nanoparticles or that are found to act in the same biofilm area, as it can happen, for example for VCN and NAC against *S. aureus* biofilms.

Therefore, the combination of two or more molecules or APIs must be carefully evaluated, as there may be a direct interaction between the molecules (chemical incompatibility) or a biological antagonism between them.

On the contrary, some combinations can lead to a synergistic effect, such as the combination of MZN loaded with and/or supporting NTF and with NAC (both in the version in which these APIs are loaded together in the same nanoparticles, and in the version in which the first API is loaded and/or supported in a first or first nanoparticles and where the second API is loaded and/or supported in a second or second nanoparticles).

This DDS system, therefore, allows to help the diffusion of at least one API within the biofilm.

Moreover, it is possible to avoid or limit the problems deriving from the chemical incompatibility of different APIs, by loading or supplying each of them in one or more respective mesoporous nanoparticles. In this case, two or more different active ingredients or pharmacologically active ingredients (API) would be provided, and each active ingredient or pharmacologically active ingredient (API) would be loaded and/or supported in one or more mesoporous nanoparticles different from the nanoparticle or nanoparticles in which the other or pharmacologically active ingredient(s) (API) is loaded/supported or loaded/supported.

It has been experimentally observed that for a better interaction between the carrier and the chosen API, or the chosen APIs, the aforementioned nanoparticles, of substantially spherical and/or irregular shape and/or with a specific surface area included in a range between 100 and 500 $m^2/g$, preferably from 150 to 250 $m^2/g$, preferably from 190 to 230 $m^2/g$, must - in at least one version of the invention - have an average pore diameter having a value greater than the size of the API, in so that most of the surface and pore volume of the MZNs are accessible by the API to facilitate their loading.

On the basis of the typical diameters for the APIs indicated above it will be preferable that the nanoparticles have pores with a diameter generally between 2 and 50 nm, but preferably between 2 and 10 nm or between 4 and 6 nm and an average pore volume substantially comprised in a range from 0.2 to 0.4 $cm^3/g$, but preferably between 0.25 and 0.35 $cm^3/g$. In particular, even for large APIs, such as vancomycin, provided that with an apparent molecular diameter lower than the average pore diameter, the API is loaded in weight% varying between 5 and 20% with respect to the weight of the particles.

**Example 1: protocol for synthesis**

The protocol used for the synthesis of MZNs is based on a sol-gel process and foresees a molar ratio of the reagents ZB: EtOH: $H_2O$: NaF: HDA pari a 1: 750: 20: 0,02: 2 (WO2016120795).

Hexadecylamine (HDA) dissolved in ethanol (EtOH) was used as surfactant, followed by the addition of a sodium fluoride solution NaF 0.1 M and MilliQ water, under stirring and at a temperature between 20 and 25°C. The precursor of zirconium butoxide (ZB) dissolved in ethanol was added dropwise in the first solution under stirring, observing that the mixture turned white due to the formation of insoluble MZNs.

Subsequently, three washing cycles were carried out in EtOH, possibly with demineralized water, recovering the particulate by centrifugation (15 minutes at 12000 rcf). The dried powder, suspended in MilliQ water and ethanol, was heated to 170°C for 20 h.

The collected sample was finally subjected to a thermal vacuum extraction and/or calcination process between 300 and 350°C to remove the residual surfactant.

In fact, therefore, the synthesis of MZNs includes a sol-gel method with neutral surfactant and the use of an inorganic salt. It is an effective process in which the neutral surfactant helps to define the pores of the MZNs, in combination with a salt to avoid aggregation, a hydrothermal treatment to define the shape of the particles and the extraction of the surfactant, by vacuum and/or high temperatures, in order to avoid the collapse of the pores.

SEM and TEM analyses showed mesoporous nanoparticles with regular shape, spheroidal appearance and controlled size. These particles are porous, well defined and well separated.

In at least one version, both the shape and the dimensions are uniform.

In another version of the invention, the particles can have an irregular shape and/or size, or a combination with the shape and/or size indicated above.

The high resolution TEM analysis showed roughness on the MZN, probably due to their porosity and partial crystallinity. Several synthesis batches have provided good reproducibility, with an average diameter of the MZNs ranging from 100 to 600 nm, or between 100 and 500 nm, preferably between 200 and 400 nm or between 200 and 300 nm or from 150 to 350 nm.

The presence of surface charges on the s influences the colloidal stability of the nanoparticles and is also dependent on the pH of the solution. Furthermore, the surface charge properties of the vector are important properties to consider in investigating both the loading and release processes of the different APIs.

The surface charge of the MZN was estimated as a function of the pH of the solution. Under acidic conditions at low pH values, the ZP of the MZN was positive (25-30 mV). The number of positive surface charges decreases at higher pH values, with the isoelectric point falling within the range of pH 6.0 to 7.0. Beyond this point the ZP is negatively charged, reaching a plateau of about -30 mV at pH = 10.0.

DLS (*Dynamic Light Scattering*) measurements carried out in water at neutral pH showed a hydrodynamic diameter of the nanoparticles from 200 to 650 nm and a polydispersion index (PdI) from 0.3 to 0.6.

**Example 2: protocol for loading**

A method for the production of the DDS described above initially involves a step of providing mesoporous zirconia nanoparticles (MZNs) and at least one active or pharmacologically active ingredient (API) comprising at least one substance with antibacterial, bacteriostatic and/or bactericidal activity and/or mucolytic and/or anti-inflammatory. Then an impregnation step of the MZNs follows, by dispersing a predetermined quantity of said MZN in water or organic solvent, based on the specific solubility of the API chosen, containing a predetermined quantity of at least one API by obtaining a suspension, subsequent mixing of the suspension with the resulting loading and/or absorption and/or support of the at least one active principle or pharmacologically active ingredient (API) in the pores and/or on the external surface and/or in the inner structure of the MZNs, and a step of separation of the nanoparticles loaded with API through centrifugation or filtration.

The loading step can involve the insertion of the at least one active principle or pharmacologically active ingredient (API) into the pores of the mesoporous zirconia nanoparticles (MZN). This API, as mentioned, can however also be adsorbed or supported by the MZN and/or by their external surface.

For example, in accordance with the solubility of the API, the NAC or the VCN, have been loaded and/or supported starting from aqueous solutions with a known title of API. The loading efficiency was calculated by UV-Vis and quantitative HPLC on the supernatant solution expressed as the ratio between the weight of the loaded and/or supported API and the quantity of MZN.

In the loading protocols used, after each loading, the supernatant solution was separated thus determining the quantity of active ingredient actually loaded or encapsulated. The results and loading conditions are shown in Table 1.

| API | Solvent/buffer | Solubility H₂O (mg/mL) | LogP* | Experimental loading efficiency (%) | Model 1 Max. loading (%) | Model 2 Max. loading $A_{Max}$(%)- $A_{Min}$(%) |
|---|---|---|---|---|---|---|
| IBU | Ethanol | 0.021 | 3.97 | 15±5 | 32 | 8-15 |
| NAC | MilliQ water | 5.09 | -0.03 | 10±3 | 39 | 8-13 |
| GNTM | MilliQ water, (acid, basic or neutral buffer) | >50 | -3.10 | 0 | 32 | 10-17 |
| NTF | DMF | 0.08 | -0.47 | 10±5 | 49 | 9-19 |
| INDM | Acetone | 0.08 | 4.27 | 10±5 | 37 | 9-19 |
| VCN | MilliQ water | >50 | -3.10 | 10±2 | 40 | 13-20 |

The loading and/or support of the active ingredient was found to be affected by several parameters, such as the solubility and polarity of the API, the solvation properties of the solvent, and the electrostatic interactions with MZNs for the charged drugs.

Among other features, the size, shape and surface area of the API as primary properties play a key role in influencing the theoretical maximum load capacity of the API.

There was good API load capacity in MZNs between 5 and 20% w/w.

For example, the loading efficiency for VCN can vary between 5-15% (weight/weight) or between 8-12% (weight/weight), for NTF between 2-20% (weight/weight) or between 5-15 % (weight/weight) while for NAC it can vary between 5-15% (weight/weight) or between 7-13% (weight/weight).

Plate antimicrobial tests were conducted to verify the inactivity of the nanoparticles and to confirm that the antibacterial activity is linked to being loaded with at least one API. The negative control corresponds to *S. aureus* and *K. pneumoniae* without API.

In particular, MZNs are completely inactive if not loaded against both strains.

NAC does not exhibit antibacterial activity below 1-2 mg/ml, being a mucolytic agent.

**Example 3: Biofilm eradication test**

The eradication tests were performed by adding free or loaded and/or supported API after the maturation of a biofilm for 24 hours.

VCN was tested against *S. aureus* and NTF against *K. pneumoniae.*

Table 2 below shows the main results regarding the Log Reduction of preformed biofilm live cells at the highest concentrations tested for each API agent.

The log reduction was calculated according to the following formula:

Log reduction (LR) = $\mathrm{Log}_{10}$(mean number of CFUs without treatment)/(mean number of CFUs after treatment), where CFUs are *Colony Formation Units.*

Correspondingly, the eradication percentage P was calculated according to the following formula:

$$P\,(\%) = [1\text{-}(10^{\text{-LR}})]*100$$

| Eradication tests | | | |
|---|---|---|---|
| | Log Reduction Log (CFU / ml)±SD | P (%) | Strain |
| **MZN** | 0.0 | / | *S. aureus* and K. pneumoniae |
| **Loaded VCN** | 3.0± 0.2 | 99.9 | *S. aureus* |
| **Free VCN** | 2.0± 0.4 | 99.0 | *S. aureus* |
| **Loaded NTF** | 4.5± 2.0 | 99.997 | *K. pneumoniae* |
| **Free NTF** | 2.5± 1.0 | 99.7 | *K. pneumoniae* |

In particular, loaded and/or supported APIs show a greater log reduction of at least 1 when compared with the same concentration of free API.

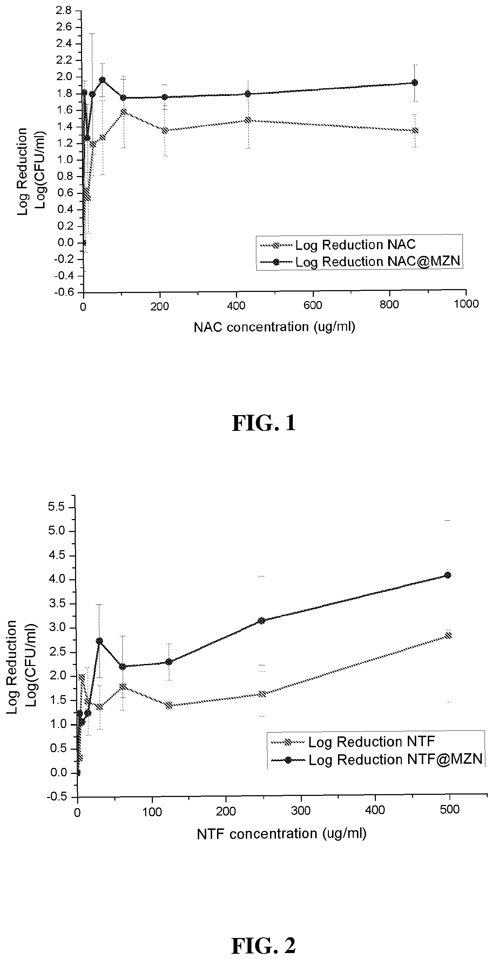

The graphs in Figures 1, 2 and 3 show the results of these experiments.

Fig. 1 shows an example of a *Staphylococcus aureus* biofilm eradication test, comparing free and loaded NAC in MZN. Testing was performed by MBEC assay with Calgary Biofilm Device. Enhanced biofilm eradication action from API when loaded.

Fig. 2 shows an example of *Klebsiella pneumoniae* biofilm eradication test, comparing free and loaded NTF in MZN. Testing was performed by MBEC assay with Calgary Biofilm Device. Enhanced biofilm eradication action from API when loaded.

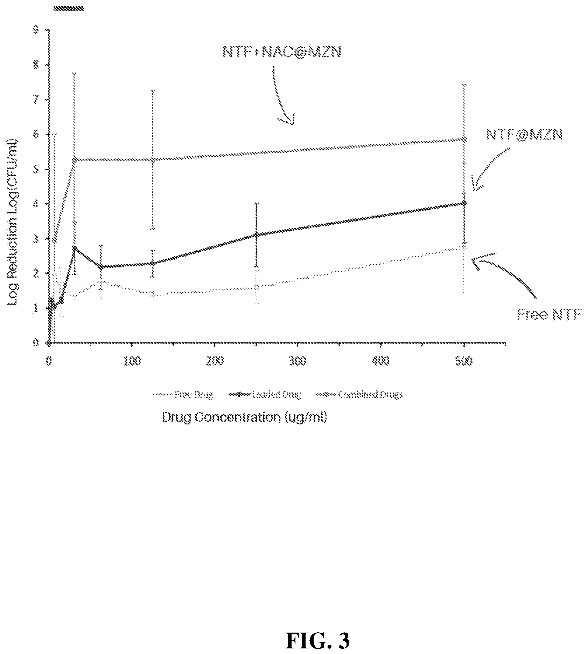

Finally, Fig. 3 shows an example of a synergistic effect in terms of eradication of *K. pneumoniae* biofilms of two APIs supported by MZN. The result of the combination (during the biofilm eradication process) of MZN loaded with and/or supporting NAC and MZN loaded with and/or supporting NTF shows a synergistic action for the eradication of a *K. pneumoniae* biofilm, better than eradication by NTF loaded on MZN (NTF@MZN) or by free NTF.

In general, therefore, the loading process on MZN allows to mix APIs with different solubilities and behaviours, such as NTF and NAC in the proposed example, obtaining a better eradication of the biofilm.

## Claims

1. A system for the administration and delivery of at least one active principle for the eradication of an infection or contamination, wherein said infection or contamination is caused by a bacterial biofilm, comprising:

   - a vector or carrier selected from the mesoporous nanoparticles comprising pores;
   - at least one active principle or pharmacologically active ingredient (API) loaded into said pores of said vector and/or supported by and/or adsorbed in said vector;

   wherein said vector is constituted by mesoporous zirconia nanoparticles (MZNs), wherein said zirconia of said MZNs has high affinity with phosphate groups whereby said MZNs interact directly with the bacterial cell wall of said biofilm and wherein said API includes N-acetyl-L-cysteine (NAC).

2. System according to claim 1, wherein NAC is combined with least one other active principle or pharmacologically active ingredient (API) selected from the group of molecules with antimicrobial and/or antibacterial and/or mucolytic and/or anti-inflammatory activity, more specifically comprising at least one antibiotic, vancomycin, nitrofurantoin.

3. System according to claim 1, wherein said nanoparticles have a substantially spherical and/or spheroidal and/or irregular shape and/or an average surface area comprised in a range between 100 and 500 $m^2/g$, between 150 and 250 $m^2/g$, or between 190 and 230 $m^2/g$ and/or an average diameter in the range between 100 and 600 nm, or between 100 and 500 nm, or between 200 and 400 nm or between 200 and 300 nm or between 150 and 350 nm.

4. System according to any one of the preceding claims, wherein said system has a load capacity of said at least one active principle or pharmacologically active ingredient (API) in said mesoporous zirconia nanoparticles comprised between 5 and 20% (weight/weight).

5. System according to any one of the preceding claims, wherein said vector or carrier has an internal structure and an external surface and said pores are present in said internal structure and/or on said external surface and/or wherein said pores have a diameter between 2 and 50 nm, between 2 nm and 10 nm or between 4 nm and 6 nm and/or wherein said pores have an average volume substantially comprised in a range from 0.2 to 0.4 $cm^3/g$, or between 0.25 and 0.35 $cm^3/g$.

6. System according to claim 5, wherein said MZNs have a positive ZP under acidic conditions at low pH values, for example of 25-30 mV, and have isoelectric point falling within the range of pH 6.0 to 7.0.

7. System according to claim 2, comprising two or more active principles or pharmacologically active ingredients (API) different from each other, and wherein each active principle or pharmacologically active ingredient (API) is loaded in one or more mesoporous nanoparticles different from the nanoparticle or nanoparticles into which the other active principle(s) or pharmacologically active ingredient(s) (API) is loaded or are loaded.

8. System according to claim 7, wherein vancomycin or nitrofurantoin is loaded and/or supported in one or more first mesoporous nanoparticles, while N-acetyl-L-cysteine (NAC) is loaded and/or supported in other or second mesoporous nanoparticles different from the first.

9. Method for the production of a system for the delivery and administration of at least one active principle for the eradication of an infection or contamination, wherein said infection or contamination is caused by bacterial biofilm according to one or more of the preceding claims, comprising:

a step of providing mesoporous zirconia nanoparticles (MZNs), wherein said zirconia of said MZNs has high affinity with phosphate groups whereby said MZNs interact directly with the bacterial cell wall of said biofilm, and a step of providing at least one active principle or pharmacologically active ingredient (API) and wherein said API includes N-acetyl-L-cysteine (NAC), a step of impregnation of said mesoporous zirconia nanoparticles (MZNs) by dispersing a predetermined quantity of said MZNs in water or organic solvent containing a predetermined quantity of said at least one active principle or pharmacologically active ingredient (API) with the formation of a suspension, subsequent mixing of the suspension with consequent loading of said at least one active principle or pharmacologically active ingredient (API) into the pores of said mesoporous zirconia nanoparticles (MZNs) and/or support and/or adsorption of said at least one active principle or pharmacologically active ingredient (API) in said mesoporous zirconia nanoparticles (MZNs) and, a step of separating said nanoparticles loaded with said at least one active principle or pharmacologically active ingredient (API) by means of centrifugation or filtration.

10. Method according to claim 9, wherein said NAC is combined with at least one active principle or pharmacologically active ingredient (API) selected from the group of molecules with antimicrobial and/or antibacterial and/or mucolytic and/or anti-inflammatory activity, more specifically comprising at least one antibiotic, vancomycin, nitrofurantoin.

11. Method according to claim 9 or 10, wherein said loading step provides for the insertion of said at least one active principle or pharmacologically active ingredient (API) in said pores of said mesoporous zirconia nanoparticles (MZNs) and/or the adsorption of said at least one active principle or pharmacologically active ingredient (API) in said mesoporous zirconia nanoparticles (MZNs) and/or on the external surface of said mesoporous zirconia nanoparticles (MZNs).

12. Method according to any one of claims 9 to 11, wherein said loading step has a loading capacity of said at least one active principle or pharmacologically active ingredient (API) in said mesoporous zirconia nanoparticles comprised between 5 and 20% (weight/weight).

13. System according to one or more of claims 1 to 8, for use in the eradication of an infection or contamination, wherein said infection or contamination is caused by a bacterial biofilm.

14. System for use according to claim 13, wherein the antibiofilm activity specifically of eradication is enhanced by the loading and/or supporting process of said at least one active principle or pharmacologically active ingredient (API) via said mesoporous zirconia nanoparticles and by the vehicle of said at least one active principle or pharmacologically active ingredient (API) through said mesoporous zirconia nanoparticles in proximity and/or near and/or inside said biofilm.

**Patentansprüche**

1. System zur Verabreichung und Abgabe von mindestens einem Wirkstoff zur Ausrottung einer Infektion oder Kontamination, worin die besagte Infektion oder Kontamination durch einen bakteriellen Biofilm verursacht wird, umfassend:

- einen Vektor oder Träger, ausgewählt aus den mesoporösen Nanopartikeln, umfassend Poren;
- mindestens einen Wirkstoff oder pharmakologisch wirksamen Bestandteil (API), der in die besagten Poren des besagten Vektors geladen wird und/oder von dem besagten Vektor getragen und/oder in diesen adsorbiert wird;

worin der besagte Vektor aus mesoporösen Zirkoniumdioxid-Nanopartikeln (MZN) besteht, worin das besagte Zirkoniumdioxid der besagten MZN eine hohe Affinität zu Phosphatgruppen aufweist, wodurch die besagten MZN direkt mit der bakteriellen Zellwand des besagten Biofilms interagieren, und worin der besagte API N-Acetyl-L-Cystein (NAC) beinhaltet.

2. System nach Anspruch 1, worin NAC mit mindestens einem anderen Wirkstoff oder pharmakologisch wirksamen Bestandteil (API) kombiniert ist, der aus der Gruppe der Moleküle mit antimikrobieller und/oder antibakterieller und/oder mukolytischer und/oder entzündungshemmender Wirkung ausgewählt ist, insbesondere umfassend mindestens ein Antibiotikum, Vancomycin, Nitrofurantoin.

3. System nach Anspruch 1, worin die besagten Nanopartikel eine im Wesentlichen sphärische und/oder sphäroidische und/oder unregelmäßige Form und/oder eine durchschnittliche Oberfläche, die in einem Bereich zwischen 100 und 500 $m^2/g$, zwischen 150 und 250 $m^2/g$ oder zwischen 190 und 230 $m^2/g$ liegt, und/oder einen durchschnittlichen Durchmesser, der im Bereich zwischen 100 und 600 nm oder zwischen 100 und 500 nm oder zwischen 200 und 400 nm oder zwischen 200 und 300 nm oder zwischen 150 und 350 nm liegt, aufweisen.

4. System nach irgendeinem der vorangegangenen Ansprüche, worin das besagte System eine Beladungskapazität des besagten mindestens einen Wirkstoffs oder pharmakologisch wirksamen Bestandteils (API) in den besagten mesoporösen Zirkoniumdioxid-Nanopartikeln aufweist, die zwischen 5 und 20% (Gewicht/Gewicht) liegt.

5. System nach irgendeinem der vorangegangenen Ansprüche, worin der besagte Vektor oder Träger eine innere Struktur und eine äußere Oberfläche aufweist und die besagten Poren in der besagten inneren Struktur und/oder auf der besagten äußeren Oberfläche vorhanden sind und/oder worin die besagten Poren einen Durchmesser zwischen 2 und 50 nm, zwischen 2 nm und 10 nm oder zwischen 4 nm und 6 nm aufweisen und/oder worin die besagten Poren ein durchschnittliches Volumen aufweisen, das im Wesentlichen in einem Bereich von 0,2 bis 0,4 $cm^3/g$ oder zwischen 0,25 und 0,35 $cm^3/g$ liegt.

6. System nach Anspruch 5, worin die besagten MZN ein positives ZP unter sauren Bedingungen bei niedrigen pH-Werten, zum Beispiel von 25-30 mV, aufweisen und einen isoelektrischen Punkt, der in den Bereich von pH 6,0 bis 7,0 fällt, aufweisen.

7. System nach Anspruch 2, umfassend zwei oder mehr Wirkstoffe oder pharmakologisch wirksame Bestandteile (API), die sich voneinander unterscheiden, und worin jeder Wirkstoff oder pharmakologisch wirksame Bestandteil (API) in einem oder mehreren mesoporösen Nanopartikeln geladen ist, die sich von dem Nanopartikel oder den Nanopartikeln unterscheiden, in die der/die andere(n) Wirkstoff(e) oder pharmakologisch wirksame(n) Bestandteil(e) (API) geladen ist/sind.

8. System nach Anspruch 7, worin Vancomycin oder Nitrofurantoin in einem oder mehreren ersten mesoporösen Nanopartikeln geladen und/oder getragen wird, während N-Acetyl-L-Cystein (NAC) in anderen oder zweiten mesoporösen Nanopartikeln, die sich von den ersten unterscheiden, geladen und/oder getragen wird.

9. Verfahren zur Herstellung eines Systems zur Abgabe und Verabreichung von mindestens einem Wirkstoff zur Ausrottung einer Infektion oder Kontamination, worin die besagte Infektion oder Kontamination durch einen bakteriellen Biofilm nach einem oder mehreren der vorangegangenen Ansprüche verursacht wird, umfassend:

einen Schritt des Bereitstellens mesoporöser Zirkoniumdioxid-Nanopartikel (MZN), worin das besagte Zirkoniumdioxid der besagten MZN eine hohe Affinität zu Phosphatgruppen aufweist, wodurch die besagten MZN direkt mit der bakteriellen Zellwand des besagten Biofilms interagieren, und
einen Schritt des Bereitstellens mindestens eines Wirkstoffs oder pharmakologisch wirksamen Bestandteils (API), und worin der besagte API N-Acetyl-L-Cystein (NAC) beinhaltet,
einen Schritt der Imprägnierung der besagten mesoporösen Zirkoniumdioxid-Nanopartikel (MZN) durch Dispergieren einer vorbestimmten Menge der besagten MZN in Wasser oder organischem Lösungsmittel, das eine vorbestimmte Menge des besagten mindestens einen Wirkstoffs oder pharmakologisch wirksamen Bestandteils (API) enthält, mit der Bildung einer Suspension,
anschließendes Mischen der Suspension mit nachfolgender Beladung des besagten mindestens einen Wirkstoffs oder pharmakologisch wirksamen Bestandteils (API) in die Poren der besagten mesoporösen Zirkoniumdioxid-Nanopartikel (MZN) und/oder Tragen und/oder Adsorption des besagten mindestens einen Wirkstoffs oder pharmakologisch wirksamen Bestandteils (API) in den besagten mesoporösen Zirkoniumdioxid-Nanopartikeln (MZN) und,
einen Schritt des Trennens der besagten Nanopartikel, die mit dem besagten mindestens einen Wirkstoff oder pharmakologisch wirksamen Bestandteil (API) mittels Zentrifugation oder Filtration beladen sind.

10. Verfahren nach Anspruch 9, worin das besagte NAC mit mindestens einem Wirkstoff oder pharmakologisch wirksamen Bestandteil (API) kombiniert ist, der aus der Gruppe der Moleküle mit antimikrobieller und/oder antibakterieller und/oder mukolytischer und/oder entzündungshemmender Wirkung ausgewählt ist, insbesondere umfassend mindestens ein Antibiotikum, Vancomycin, Nitrofurantoin.

11. Verfahren nach Anspruch 9 oder 10, worin der besagte Beladungsschritt das Einbringen des besagten mindestens einen Wirkstoffs oder pharmakologisch wirksamen Bestandteils (API) in die besagten Poren der besagten mesoporösen Zirkoniumdioxid-Nanopartikel (MZN) und/oder die Adsorption des besagten mindestens einen Wirkstoffs oder pharmakologisch wirksamen Bestandteils (API) in den besagten mesoporösen Zirkoniumdioxid-Nanopartikeln (MZN) und/oder auf der äußeren Oberfläche der besagten mesoporösen Zirkoniumdioxid-Nanopartikel (MZN) vorsieht.

12. Verfahren nach irgendeinem der Ansprüche 9 bis 11, worin der besagte Beladungsschritt eine Beladungskapazität des besagten mindestens einen Wirkstoffs oder pharmakologisch wirksamen Bestandteils (API) in den mesoporösen Zirkoniumdioxid-Nanopartikeln aufweist, die zwischen 5 und 20% (Gewicht/Gewicht) liegt.

13. System nach einem oder mehreren der Ansprüche 1 bis 8, zur Verwendung bei der Ausrottung einer Infektion oder Kontamination, worin die besagte Infektion oder Kontamination durch einen bakteriellen Biofilm verursacht wird.

14. System zur Verwendung nach Anspruch 13, worin die Antibiofilm-Aktivität insbesondere zur Ausrottung durch den Beladungs- und/oder Tragprozess des besagten mindestens einen Wirkstoffs oder pharmakologisch wirksamen Bestandteils (API) über die besagten mesoporösen Zirkoniumdioxid-Nanopartikel und durch das Vehikel des besagten mindestens einen Wirkstoffs oder pharmakologisch wirksamen Bestandteils (API) durch die besagten mesoporösen Zirkoniumdioxid-Nanopartikel in der Nähe und/oder innerhalb des besagten Biofilms verstärkt wird.

**Revendications**

1. Système pour l'administration et la délivrance d'au moins un principe actif pour l'éradication d'une infection ou d'une contamination, dans lequel ladite infection ou contamination est causée par un biofilm bactérien, comprenant :

   - un vecteur ou porteur sélectionné parmi les nanoparticules mésoporeuses composées de pores ;
   - au moins un principe actif ou un ingrédient actif pharmacologiquement (API) chargé dans lesdites pores dudit vecteur et/ou supporté et/ou adsorbé dans ledit vecteur ;

   dans lequel ledit vecteur est constitué de nanoparticules de zircone mésoporeuses (MZN), dans lequel ladite zircone desdites MZN présente une forte affinité avec les groupes phosphates, moyennant quoi lesdites MZN interagissent directement avec la paroi cellulaire bactérienne dudit biofilm et dans lequel ledit API inclut N-acétyl-L-cystéine (NAC).

2. Système selon la revendication 1, dans lequel la NAC est combinée avec au moins un autre principe actif ou ingrédient actif pharmacologiquement (API) sélectionné parmi le groupe de molécules ayant une activité antimicrobienne et/ou antibactérienne et/ou mucolytique et/ou anti-inflammatoire, comprenant plus précisément au moins un antibiotique, la vancomycine, la nitrofurantoïne.

3. Système selon la revendication 1, dans lequel lesdites nanoparticules ont une forme sensiblement sphérique et/ou sphéroïdale et/ou irrégulière et/ou une surface moyenne comprise dans une plage entre 100 et 500 $m^2$/g, entre 150 et 250 $m^2$/g, ou entre 190 et 230 $m^2$/g et/ou un diamètre moyen dans la plage comprise entre 100 et 600 nm, ou entre 100 et 500 nm, ou entre 200 et 400 nm, ou entre 200 et 300 nm, ou entre 150 et 350 nm.

4. Système selon l'une des revendications précédentes, dans lequel ledit système a une capacité de charge dudit au moins un principe actif ou ingrédient actif pharmacologiquement (API) dans lesdites nanoparticules mésoporeuses de zircone composées entre 5 et 20 % (poids/poids).

5. Système selon l'une des revendications précédentes, dans lequel ledit vecteur ou porteur possède une structure interne et une surface externe, et lesdits pores sont présents dans ladite structure interne et/ou sur ladite surface externe et/ou dans lequel lesdits pores ont un diamètre compris entre 2 et 50 nm, entre 2 nm et 10 nm ou entre 4 nm et 6 nm et/ou dans lequel lesdits pores ont un volume moyen sensiblement compris dans une plage entre 0,2 et 0,4 $cm^3$/g, ou entre 0,25 et 0,35 $cm^3$/g.

**6.** Système selon la revendication 5, dans lequel lesdites MZN ont un ZP positif sous conditions acides à de faibles valeurs de pH, par exemple de 25-30 mV, et ont un point isoélectrique se situant dans la plage de pH 6,0 à 7,0.

**7.** Système selon la revendication 2, composé de deux ou plusieurs principes actifs ou ingrédients actifs pharmacologiques (API) différents entre eux, et dans lequel chaque principe actif ou ingrédient actif pharmacologiquement actif (API) est chargé dans une ou plusieurs nanoparticules mésoporeuses différentes de la nanoparticule ou des nanoparticules dans lesquelles le(s) autre(s) principe(s) actif(s) ou ingrédient(s) pharmaceutiquement actif(s) (API) est ou sont chargé(s).

**8.** Système selon la revendication 7, dans lequel la vancomycine ou la nitrofurantoïne est chargée et/ou supportée dans une ou plusieurs premières nanoparticules mésoporeuses, tandis que la N-acétyl-L-cystéine (NAC) est chargée et/ou supportée dans d'autres ou secondes nanoparticules mésoporeuses différentes des premières.

**9.** Procédé pour la production d'un système pour la délivrance et l'administration d'au moins un principe actif pour l'éradication d'une infection ou d'une contamination, dans lequel ladite infection ou contamination est causée par un biofilm bactérien selon une ou plusieurs des revendications précédentes, comprenant :

une étape de fournir des nanoparticules de zircone mésoporeuses (MZN), dans lequel ladite zircone desdites MZN présente une forte affinité avec les groupes phosphates, moyennant quoi lesdites MZN interagissent directement avec la paroi cellulaire bactérienne dudit biofilm, et
une étape de fournir au moins un principe actif ou un ingrédient actif pharmacologiquement (API), et dans lequel ledit API inclut N-acétyl-L-cystéine (NAC),
une étape d'imprégnation desdites nanoparticules mésoporeuses de zircone (MZN) en dispersant une quantité prédéterminée desdites MZN dans de l'eau ou un solvant organique contenant une quantité prédéterminée dudit au moins un principe actif ou principe actif pharmacologiquement (API) avec la formation d'une suspension, le mélange ultérieur de la suspension avec la charge conséquente dudit au moins un principe actif ou ingrédient actif pharmacologiquement (API) dans les pores desdites nanoparticules mésoporeuses de zircone (MZN) et/ou le support et/ou l'adsorption dudit au moins un principe actif ou ingrédient actif pharmacologiquement (API) dans lesdites nanoparticules mésoporeuses de zircone (MZN) et,
une étape de séparer lesdites nanoparticules chargées d'au moins un principe actif ou ingrédient actif pharmacologiquement (API) par centrifugation ou filtration.

**10.** Procédé selon la revendication 9, dans lequel ladite NAC est combinée avec au moins un principe actif ou un ingrédient actif pharmacologiquement (API) sélectionné parmi le groupe de molécules ayant une activité antimicrobienne et/ou antibactérienne et/ou mucolytique et/ou anti-inflammatoire, comprenant plus précisément au moins un antibiotique, la vancomycine, la nitrofurantoïne.

**11.** Procédé selon la revendication 9 ou 10, dans lequel ladite étape de chargement prévoit l'insertion dudit au moins un principe actif ou ingrédient actif pharmacologiquement (API) dans lesdits pores desdites nanoparticules mésoporeuses de zircone (MZN) et/ou l'adsorption dudit au moins un principe actif ou ingrédient actif pharmacologiquement (API) dans lesdites nanoparticules mésoporeuses de zircone (MZN) et/ou sur la surface externe desdites nanoparticules mésoporeuses de zircone (MZN).

**12.** Procédé selon l'une des revendications 9 à 11, dans lequel ladite étape de chargement a une capacité de charge dudit au moins un principe actif ou ingrédient actif pharmacologiquement (API) dans lesdites nanoparticules mésoporeuses de zircone composées entre 5 et 20 % (poids/poids).

**13.** Système selon une ou plusieurs des revendications 1 à 8, pour l'utilisation dans l'éradication d'une infection ou contamination, dans lequel ladite infection ou contamination est causée par un biofilm bactérien.

**14.** Système pour l'utilisation selon la revendication 13, dans lequel l'activité antibiofilm spécifiquement lors de l'éradication est renforcée par le processus de chargement et/ou de soutien dudit au moins un principe actif ou ingrédient actif pharmacologiquement (API) via lesdites nanoparticules mésoporeuses de zircone et par le véhicule dudit au moins un principe actif ou ingrédient actif pharmacologiquement (API) via lesdites nanoparticules mésoporeuses de zircone en proximité et/ou près et/ou à l'intérieur dudit biofilm.

**FIG. 1**

**FIG. 2**

**FIG. 3**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2016120795 A
- WO 2017041032 A
- WO 2016204896 A
- CN 101259082
- EP 2017082512 W
- WO 2018154091 A1

### Non-patent literature cited in the description

- *Advance Pharmaceutical Journal*, 2017, vol. 2 (6), 204-209
- *Pharmaceutics*, 2018, vol. 10, 279
- *Nature Materials*, 2013, vol. 12, 991-1003
- *Expert Opinion on Drug Delivery*, 2016, vol. 13 (1), 93-108
- *Pharmaceutics*, 2020, vol. 12 (5), 432
- *Molecules*, 2018, vol. 23 (1), 47
- *Nanomed Nanotechnol*, 2018, vol. 3 (2), 000136
- *Acta Biomaterialia*, 2015, vol. 13, 354-363
- *J. Mater. Chem.B*, 2015, vol. 3, 7300
- *J. Drug Deliv. Sci. Technol*, 2021, vol. 61, 102189
- *International Journal of Nanomedicine*, 2019, vol. 14, 5175-5186
- **WATER**. *Air, & Soil Pollution*, 2012, vol. 223, 4221-4231
- *Journal of the American Society for Mass Spectrometry*, 2008, vol. 19 (8), 1176-1186
- *Scientific Reports*, 2020, vol. 10, 960
- *Clinical Microbiology and Infection*, 2017, vol. 23 (6), 355-362